# EUROPEAN PATENT SPECIFICATION

(11) **EP 3 031 808 B1**
(45) Date of publication and mention of the grant of the patent: **27.06.2018**
(21) Application number: 14196931.1
(22) Date of filing: 09.12.2014
(51) Int. Cl.: C07D 473/34, A61K 31/52, A61P 35/00

(54) **SALT OF IDELALISIB**
SALZ VON IDELALISIB
SEL D'IDELALISIB

(43) Date of publication of application: 15.06.2016
(73) Proprietor: ratiopharm GmbH, 89079 Ulm (DE)
(72) Inventor: Striegel, Hans Günther, 89143 Blaubeuren (DE)
(74) Representative: Best, Michael

(56) References cited:
- WO-A1-2013/134288

## Description

The present invention relates to the maleic acid salt of idelalisib, polymorphs of this salt, methods for its preparation, and pharmaceutical preparations comprising this salt.

Idelalisib is a known active pharmaceutical ingredient which blocks the effect of an enzyme called PI3K-delta. This enzyme plays a role in the growth, migration and survival of white blood cells but is overactive in blood cancers, where it enables the survival of the cancer cells. By targeting this enzyme and blocking its effects, idelalisib causes death of the cancer cells, thereby delaying or stopping the progression of the cancer.

The chemical name of idelalisib is 5-fluoro-3-phenyl-2-[(1S)-1-(9H-purin-6-ylamino)propyl]quinazolin-4(3H)-one. Idelalisib has the following chemical structure:

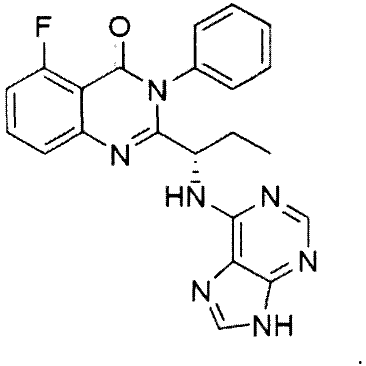

Idelalisib, its manufacture and use is described in WO 2005/113556.

Polymorphic forms of the free base of idelalisib are disclosed in WO 2013/134288.

Idelalisib is a white to off-white solid practically insoluble in water at pH 7 and soluble in water at pH 1.2. The active substance has a chiral center assigned as 1*S* and is manufactured as the pure enantiomer. The low solubility of idelalisib constitutes a problem in the formulation of pharmaceutical preparations and in bioavailability of the drug.

Therefore, there is still a need for further embodiments of idelalisib which show improved solubility and/or bioavailability. Furthermore, there is a need for embodiments of idelalisib which can be easily manufactured and which are stable under processing conditions, such as during manufacture of pharmaceutical formulations, and which are stable in a pharmaceutical formulation during storage.

It has now been found that the above and other problems can be solved by converting the free base of idelalisib into the maleic acid salt of idelalisib. Therefore, the present invention relates to the maleic acid salt of idelalisib.

One advantage of the present invention is that the maleic acid salt of idelalisib shows increased solubility compared to the free base of idelalisib.

Another advantage of the present invention is that the maleic acid salt of idelalisib can exhibit high chemical and/or physical stability both, during processing of this salt and during storage. In this context physical stability includes the stability of a certain polymorphic form against conversion into another polymorphic form.

A further advantage of the present invention is that the salt of idelalisib can exhibit low hygroscopicity which makes such salt particularly suitable for the manufacture of pharmaceutical formulations.

The maleic acid salt of idelalisib according to the invention may be crystalline, amorphous or partly crystalline.

In one embodiment the salt of idelalisib according to the invention may be a solvate, such as an ethanol, tetrahydrofuran and/or acetone solvate and/or a hydrate.

In this case, the number of solvent molecule in the crystalline structure of the salt is not limited and can be, for example, in the range of 0.1 to 2 mol per mol of idelalisib. Thus, solvates comprising for example 0.1 to 0.8 solvent molecules per idelalisib molecule are covered by the present invention.

In another embodiment, the salt of idelalisib may be desolvated.

The salt of idelalisib according to the invention is the maleic acid salt of idelalisib. This salt can be obtained in crystalline form from ethanol by evaporation and subsequent dispersion of the oily residue in ethanol/diethyl ether.

Crystalline maleic acid salt of idelalisib can be in the form of a solvate, such as a hydrate, a diethyl ether solvate and/or an ethanol solvate.

The present invention furthermore relates to a polymorphic form of the crystalline maleic acid salt of idelalisib which has an X-ray powder diffraction pattern comprising peaks at 11.7 ± 0.2, 18.2 ± 0.2, 21.0 ± 0.2, 24.0 ± 0.2 and 24.6 ± 0.2 degrees 2-theta, preferably at 10.3 ± 0.2, 11.7 ± 0.2, 12.5 ± 0.2, 13.1 ± 0.2, 14.5 ± 0.2, 15.0 ± 0.2, 16.1 ± 0.2, 17.8 ± 0.2, 18.2 ± 0.2, 19.6 ± 0.2, 20.2 ± 0.2, 21.0 ± 0.2, 24.0 ± 0.2, 24.6 ± 0.2, 26.1 ± 0.2, 26.5 ± 0.2, 26.7 ± 0.2, 27.8 ± 0.2, 28.1 ± 0.2, 28.4 ± 0.2 and 30.7 ± 0.2 degrees 2-theta.

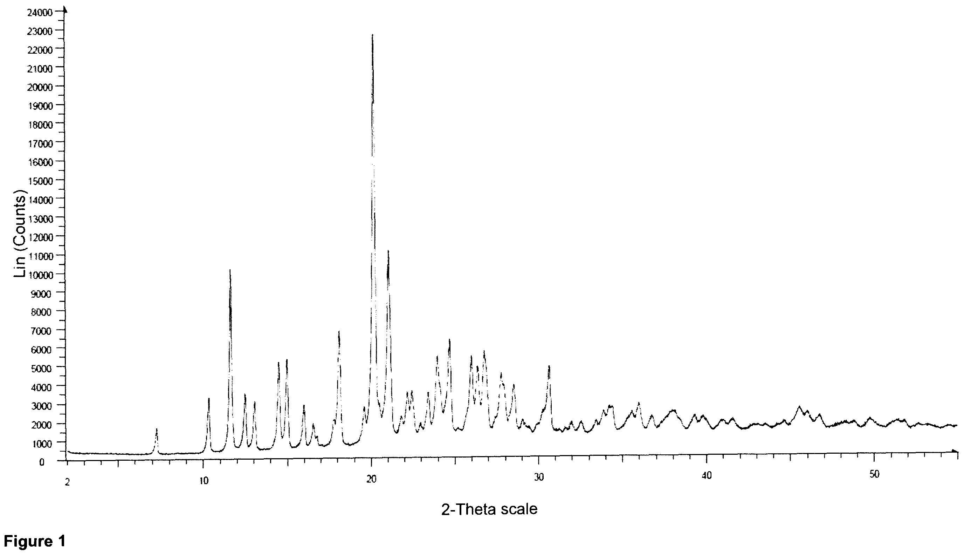

In one embodiment, the polymorphic form of crystalline maleic acid salt of idelalisib has an X-ray powder diffraction pattern of substantially as shown in figure 1.

The X-ray powder diffraction pattern of the salt of idelalisib according to the invention was measured in Bragg-Brentano-Geometry using a BrukerAXS Advance-D8 with measurement parameters as shown in the table 1.

**Table 1: conditions of measurement were as follows**

| on vertical goniometer | 435 mm measurement circle diameter | reflection, theta /2theta, |
|---|---|---|
| on 9 position sample stage | with sample rotation | 30 rpm |
| | Start angle: 2° | End Angle: 55° |
| Measurement time: | 11 min. | Step size 0.016° 2Theta |
| | | |
| Radiation: | Cu Kα1(1.5406A)/ | Tube (Siemens FLCu2K), |
| | | power 34kV/40mA |
| Monochromator: | | None |
| Detector: | position sensitive VANTEC-1 | 3° capture angle (2theta) |
| | Anti scatter slit | 6.17 mm |
| | Detector slit | 10.39 mm |
| | primary beam stop | (<2° 2theta) |
| | | 4° soller slit, |
| | Second β filter | Ni filter 0.1 mm (0.5%) |
| | | |
| Software | Diffrac^{Plus} EVA (Bruker-AXS, Karlsruhe) | release 2007 |

The invention furthermore relates to a pharmaceutical formulation comprising a salt of idelalisib as described above. Such pharmaceutical formulation may comprise one or more pharmaceutically acceptable excipients known to the skilled person. The pharmaceutical formulation may be for oral administration, such as a tablet or capsule.

The present invention furthermore relates to the above described salt of idelalisib for use in the treatment of cancer. In one embodiment, the cancer is a hematologic malignancy. The cancer may be selected from the group consisting of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disease (MPD), chronic myeloid leukemia (CML), multiple myeloma (MM), non-Hodgkin's lymphoma (iNHL), refractory iNHL, non-Hodgkin's lymphoma (NHL), mantle cell lymphoma (MCL), follicular lymphoma, Waldestrom's macroglobulinemia (WM), T-cell lymphoma, B-celllymphoma, and diffuse large B-celllymphoma (DLBCL).

Attached figure 1 shows the XRPD pattern of crystalline maleic acid salt of idelalisib.

The invention will now be explained in more detail by the following examples which are not intended to be construed as limiting.

### Reference Example 1

A screw capped sample tube, equipped with magnetic stirrer bar, was charged with 5-fluoro-3-phenyl-2-((1S)-1-((9-(tetrahydro-2H-pyran-2-yi)-9H-purin-6-yl)amino)propyl)-quinazolin-4(3H)-one (2.06 g, 0.0050 mol), absolute ethanol (2.23 g, 2.82 mL, 0.0483 mol), and water (1.41 mL, 0.0782 mol). 10N hydrochloric acid (HCl 32%) was added in portions with a Pasteur pipette (1.07 g, 0.91 mL, 0.0101 mol).

The clear to slightly turbid mixture was agitated overnight at about 21 °C but no suspension of solid hydrochloride was obtained by the procedure. The mixture remained slightly turbid. Additional absolute ethanol was added, (3.95 g, 5.00 mL 0.0858 mol) and the sample was stirred over weekend. A total amount of EtOH, 10.82 mL, 8.58 g was added, but the small amount of semisolid oily layer, which settled after ethanol addition, did not crystallize and thus could not be filtered off.

Finally the amount of dry ethanol was raised by additional 5.00 mL and stirring for 72 h completed crystallization to a filterable suspension. The filtered wet solid (2.02 g) was air dried to constant weight after 6h (1.603 g, 66%).

### Example 2

The hydrogen sulfate salt of idelalisib (not according to the invention), the nitiric acid salt of idelalisib (not according to the invention), idelalisib hydrobromide (not according to the invention), the phosphoric acid salt of idelalisib (not according to the invention), the citric acid salt of idelalisib (not according to the invention), the maleic acid salt of idelalisib, idelalisib mesylate (not according to the invention), idelalisib tosylate (not according to the invention) and idelalisib besylate (not according to the invention) and solvates thereof were prepared similar to the method described in example 1 and in the description above.

### Example 3

The solubilities of various salts of idelalisib in buffers and selected media were measured. The results are summarized in the following tables:

| | | Base | Chloride | Bromide | Hydrogen sulfate |
|---|---|---|---|---|---|
| water demin. (pH dep.) | 15 min. | 0.098 | 7.632 | 8.648 | 8.820 |
| | 60 min. | 0.081 | 17.065 | 12.102 | 8.772 |
| 20mM NaOAc pH 4.5 | 15 min. | 0.092 | 0.399 | 0.423 | 5.867 |
| | 60 min. | 0.100 | 3.274 | 1.094 | 5.982 |
| 0,01m HCl pH ∼2,2 | 15 min. | 0.947 | 17.855 | 12.460 | 9.945 |
| | 60 min. | 1.051 | 31.904 | 13.837 | 9.814 |
| 50mM KH₂PO₄ pH 6.8 | 15 min. | 0.086 | 0.353 | 0.339 | 4.961 |
| | 60 min. | 0.088 | 1.605 | 1.484 | 5.292 |
| 0.1 % TPGS (FaSSIF)* | 15 min. | 0.141 | 0.715 | 1.633 | 6.082 |
| | 60 min. | 0.107 | 3.812 | 3.481 | 6.268 |
| 2.0% TPGS (FeSSIF)* | 15 min. | 1.056 | 10.284 | 9.623 | 11.400 |
| | 60 min. | 1.172 | 14.036 | 11.168 | 11.612 |

solubilities observed for selected organic salt forms in selected buffers and media:

| | | Base | Mesylate | Tosylate | Maleate |
|---|---|---|---|---|---|
| water demin. (pH dep.) | 15 min. | 0.098 | 63.941 | 4.051 | 1.934 |
| | 60 min. | 0.081 | 62.606 | 8.607 | 2.095 |
| 20mM NaAc pH 4.5 | 15 min. | 0.092 | 37.700 | 1.088 | 0.411 |
| | 60 min. | 0.100 | 40.065 | 2.889 | 0.475 |
| 0,01m HCl pH ∼2,2 | 15 min. | 0.947 | 62.096 | 4.998 | 2.445 |
| | 60 min. | 1.051 | 63.513 | 10.301 | 2.655 |
| 50mM KH₂PO₄ pH 6.8 | 15 min. | 0.086 | 27.524 | 1.560 | 0.355 |
| | 60 min. | 0.088 | 22.953 | 3.457 | 0.393 |
| 0.1 % TPGS (FaSSIF)* | 15 min. | 0.141 | 33.818 | 2.503 | 0.662 |
| | 60 min. | 0.107 | 25.815 | 5.729 | 1.121 |
| 2.0% TPGS (FeSSIF)* | 15 min. | 1.056 | 41.337 | 7.625 | 6.798 |
| | 60 min. | 1.172 | 29.433 | 15.719 | 6.984 |

| | | | | | |
|---|---|---|---|---|---|
| TPGS: d-alpha tocopheryl polyethylene glycol 1000 succinate FaSSIF Fasted State Simulated intestinal Fluid FeSSIF Fed State Simulated Intestinal Fluid | | | | | |

## Claims

1. Maleic acid salt of idelalisib (5-fluoro-3-phenyl-2-[(1*S*)-1-(9*H*-purin-6-ylamino)propyl]quinazolin-4(3*H*)-one).

2. Salt according to claim 1 being crystalline.

3. Salt according to claim 1 being amorphous.

4. Salt according to any of the preceding claims being a solvate.

5. Salt according to claim 4, wherein the solvate is an ethanol, tetrahydrofuran, diethyl ether or acetone solvate, each of the above optionally in combination with water or a hydrate.

6. Salt according to any of claims 1 to 5 being crystalline and having an X-ray powder diffraction pattern comprising peaks at 11.7 ± 0.2, 18.2 ± 0.2, 21.0 ± 0.2, 24.0 ± 0.2 and 24.6 ± 0.2 degrees 2-theta.

7. Salt according to claim 6 having an X-ray powder diffraction pattern substantially as shown in figure 1.

8. Pharmaceutical formulation comprising a salt of idelalisib according to any of claims 1 to 7.

9. Salt of idelalisib according to any of claims 1 to 7 for use in the treatment of cancer.

## Patentansprüche

1. Maleinsäuresalz von Idelalisib (5-Fluor-3-phenyl-2-[(1*S*)-1-(9*H*-purin-6-ylamino)propyl]chinazolin-4(3*H*)-on).

2. Salz nach Anspruch 1, welches kristallin ist.

3. Salz nach Anspruch 1, welches amorph ist.

4. Salz nach einem der vorstehenden Ansprüche, welches ein Solvat ist.

5. Salz nach Anspruch 4, wobei das Solvat ein Ethanol-, Tetrahydrofuran-, Diethylether- oder Acetonsolvat ist, jedes der obigen gegebenenfalls in Kombination mit Wasser oder einem Hydrat.

6. Salz nach einem der Ansprüche 1 bis 5, welche kristallin ist und ein Röntgenpulverbeugungsmuster hat, das Peaks bei 11,7 ± 0,2, 18,2 ± 0,2, 21,0 ± 0,2, 24,0 ± 0,2 und 24,6 ± 0,2 Grad 2-Theta umfasst.

7. Salz nach Anspruch 6 mit einem Röntgenpulverbeugungsmuster, wie im Wesentlichen in Figur 1 gezeigt.

8. Pharmazeutische Formulierung, umfassend ein Salz von Idelalisib nach einem der Ansprüche 1 bis 7.

9. Salz von Idelalisib nach einem der Ansprüche 1 bis 7 zur Verwendung bei der Behandlung von Krebs.

## Revendications

1. Sel d'acide maléique d'idélalisib (5-fluoro-3-phényl-2-[(1S)-1-(9H-purin-6-ylamino)propyl]quinazolin-4(3H)-one).

2. Sel selon la revendication 1 qui est cristallin.

3. Sel selon la revendication 1 qui est amorphe.

4. Sel selon l'une quelconque des revendications précédentes qui est un solvate.

5. Sel selon la revendication 4, dans lequel le solvate est un éthanol, un tétrahydrofuranne, un éther de diéthyle ou un solvate d'acétone, chacun de ceux-ci étant éventuellement en combinaison avec de l'eau ou un hydrate.

6. Sel selon l'une quelconque des revendications 1 à 5 qui est cristallin et présente un motif de diffraction de rayons X sur poudre comprenant des pics à 11,7 ± 0,2, 18,2 ± 0,2, 21,0 ± 0,2, 24,0 ± 0,2 et 24,6 ± 0,2 degrés 2-thêta.

7. Sel selon la revendication6, ayant un motif de diffraction de rayons X sur poudre sensiblement comme montré sur la Fig. 1.

8. Formulation pharmaceutique comprenant un sel d'idélalisib selon l'une quelconque des revendications 1 à 7.

9. Sel d'idélalisib selon l'une quelconque des revendications 1 à 7 pour utilisation dans le traitement du cancer.
